(19) Europäisches Patentamt
European Patent Office
Office européen des brevets

(11) Publication number: **0 155 737**
**A2**

(12) # EUROPEAN PATENT APPLICATION

(21) Application number: 85200398.7

(22) Date of filing: 18.03.85

(51) Int. Cl.[4]: A 61 K 7/08
C 11 D 1/94

(30) Priority: 21.03.84 JP 55019/84

(43) Date of publication of application: 25.09.85 Bulletin 85/39

(84) Designated Contracting States: CH DE FR LI

(71) Applicant: SHIONOGI & CO., LTD.
12, Dosho-machi 3-chome Higashi-ku
Osaka 541(JP)

(72) Inventor: Ukita, Hisako
5-10-7, Uematsu-cho
Yao-shi Osaka(JP)

(72) Inventor: Kawai, Sadao
1-15-7, Kawai
Matsubara-shi Osaka(JP)

(72) Inventor: Egawa, Shohei
2-4-15, Nanatsumatsu-cho
Amagasaki-shi Hyogo(JP)

(74) Representative: Hranitzky, Wilhelm Max et al,
NOVAPAT - CABINET CHEREAU 5, Place du Molard
CH-1204 Genève(CH)

(54) **Low irritating shampoo compositions.**

(57) The present invention relates to low irritating shampoo compositions containing (a) a betaine, (b) anionic surfactants consisting of (1) triethanolamine laurylsulfate and (2) one or more anionic surfactants selected from the group consisting of alkali metal alkylsulfates, N-acylamino acids or the salts thereof and polyoxyethylene alkyl ether sulfates and (c) a nonionic sufactant at a weight ratio of about 1:2.5-4:0.6-1 or about 1:1:2.6-3 being suitable for patients with dermatitis, particularly atopic dermatitis.

EP 0 155 737 A2

0155737

# LOW IRRITATING SHAMPOO COMPOSITIONS

High-lathering and non-irritating detergent compositions have been disclosed in U.S. Patent specification No. 3,950,417. The compositions comprise a betaine, an anionic surfactant and a water-soluble polyoxyethylene derivative of a hydrophobic base at a weight ratio of about 1:1:3. The anionic surfactant to be used in the compositions is not, however, definitely limited; namely it does not characterize the detergent composition. Therefore, the above specification discloses none of the shampoo compositions of this invention comprising (a) a betaine, (b) at least two kinds of anionic surfactants selected from triethanolamine laurylsulfate and other specified anionic surfactants and (c) a nonionic surfactant at a specified ratio, being applicable to patients with dermatitis because of the low irritation.

The present invention relates to low irritating shampoo compositions containing (a) a betaine, (b) anionic surfactants consisting of (1) a triethanolamine laurylsulfate and (2) one or more species of anionic surfactants selected from the group consisting of alkali metal alkylsulfates, N-acylamino acids or the salts thereof and polyoxyethylene alkyl ether sulfates and (c) a nonionic surfactant at a specified ratio being applicable to patients with dermatitis, especially atopic dermatitis.

Shampoo compositions are required to have the following properties; (1) high cleaning effect, (2) stable high-lathering potency and (3) making hair lustrous and supple after shampooing. There have been many shampoos on the market of which components and the component ratio are designed in various manner to make them possess the above properties.

Low irritating medicated shampoos for use by children and anaphylactic persons are commercially available. Any shampoo, however, which patients with dermatitis, especially patients with atopic dermatitis can use with satisfaction is not found in the market at present. When a shampoo having insufficient cleaning effect is applied to patients with dermatitis, especially patients with atopic dermatitis, it is difficult to remove completely soil which causes the dermatitis worse. On the other hand, when a shampoo with strong cleaning effect is applied to these patients, their dermatitis on the scalp becomes worse with irritation and the scalp skin is defated excessively so that the skin is too dried and itching increases. As the result, the dermatitis becomes worse. Inspite of strong desire in the medical and sanitary fields, the satisfactory shampoo for the patients has not been available.

It has been discovered that shampoo compositions comprising an amphoteric surfactant betaine, two or more kinds of anionic surfactants and one or more kinds of nonionic surfactants at a specified ratio can be satisfactorily used by patients with dermatitis without getting the dermatitis worse and, additionaly, with

supressing the irritation.

The shampoo compositions of the present invention comprises the following components:

a. a betaine 1-12 % by weight

b. anionic surfactants consisiting of

(1) triethanolamine laurylsulfate 3-30 % by weight, and

(2) one or more anionic surfactants selected from the group consisting of alkali metal alkylsulfates, N-acylamino acids or its salts and polyoxyethylene alkyl ether sulfates 1-10 % by weight; and,

c. one or more nonionic surfactants selected from fatty acid alkanolamides and water-soluble polyoxyethylene derivatives 1-36 % by weight

at a weight ratio of 1:2.5-4:0.6-1, preferably about 1:3:1, or 1:1:2.6-3.

The composition contains the above components with water and may also include conditioning agents, humectant, preservatives, hair growth stimulants, fragrances, coloring agents, pearl agents, pH adjuster and the like.

The combination of the anionic surfactants are illustrated below:

triethanolamine laurylsulfate and

(1) an alkali metal alkylsulfate,

(2) a N-acylamino acid or its salt,

(3) a polyoxyethylene alkyl ether sulfate

(4) an alkali metal alkylsulfate and a N-acylamino acid or its

salt,

(5) an alkali metal alkylsulfate and polyoxyethylene alkyl ether sulfate, or

(6) an alkali metal alkylsulfate, a N-acylamino acid or its salt and a polyoxyethylene alkyl ether sulfate

Besides, the ratio of N-acylamino acid or its salt, one of the anionic surfactants to be added, is preferably controlled below 5 % by weight to the whole amount of the composition since the viscosity of the composition becomes lower when the ratio is high.

The shampoo composition of about 1:2.5-4:0.6-1 comprises about 2-8 % by weight of a betaine, about 5-32 % by weight of two or more anionic surfactants and about 1.2-8 % by weight of one or more nonionic surfactants to the whole amount of the composition and that of about 1:1:2.6-3 comprises about 4-10 % by weight of a betaine, about 4-10 % by weight of two or more aninonic surfactants and about 10.4-30 % by weight of one or more nonionic surfactants to the whole amount of the composition. The preferable one of about 1:3:1 comprises about 2-8 % by weight of a betaine, about 6-24 % by weight of two or more anionic surfactants and about 2-8 % by weight of one or more nonionic surfactants to the whole amount of the composition.

The betaine used in the present invention is represented by the formula:

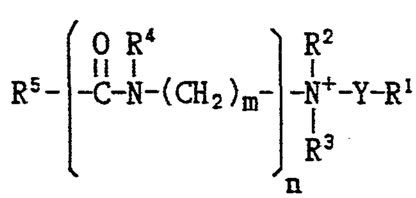

wherein $R^1$ is $COO^-$, $CH_2CH_2SO_3^-$ or $CH(OH)-CH_2SO_3^-$; $R^2$ and $R^3$ are the same or different lower alkyl; $R^4$ is hydrogen or lower alkyl; $R^5$ is higher alkyl; Y is lower alkylene; n is either 0 or 1, and m is an integer of from 2 to 7.

The betaine to be used in the present invention is, for example, an alkyl betaine (e.g. lauryldimethylammonioacetate, decyldimethylammonioacatate, cocodimethylammonioacetate (CIFA: Cocobetaine)), an alkylsulfo betaine (e.g. cocodimethylammoniopropylsulfonate(CIFA: Cocosultaine)), an alkylamido betaine (e.g.cocamidoethyldimethylammonioacetate (CIFA: Cocamidoethyl betaine), cocamidopropyldimethylammonioacetate (CIFA: Cocamidopropyl betaine), laurylamidopropyldimethylammonioacetate (CIFA: Lauramidopropyl betaine), cocamidopropyldimethylammonioacetate (CIFA: Cocamidopropyl betaine)), an alkylamidosulfo betaine (e.g. cocamidopropyldimethylammoniohydroxypropylsulfonate (CIFA:Cocamidopropyl hydroxysultaine) or the like. The names noted with CIFA in the above parentheses are the compound names described in CIFA; Cosmetic Ingredient Dictionary (3rd edition) published by the Cosmetic, Toiletry and Fragrance Association in the U.S.A.

The betaine preferably used are lauryldimethylammonioacetate, cocamidopropyldimethylammonioacetate and cocamidopropyldimethylammoniohydroxypropylsulfonate.

The alkali metal alkylsulfate to be used in the present invention is represented by the formula:

$$R^6OSO_3M$$

wherein $R^6$ is $C_{12}$-$C_{18}$ alkyl and M is sodium or potassium.

Lauryl, myristyl, cetyl and stearyl are exemplified as $R^6$. There are exemplified as alkali metal alkylslfate, sodium laurylsulfate, potassium laurylsulfate, sodium myristylsulfate, sodium cetylsulfate, sodium stearylsulfate and the like. Sodium laurylsulfate is prefeably used.

The N-acylamino acid and its salts to be used in the present invention are represented by the formula:

$$R^6-\overset{\overset{\large O}{||}}{C}-\overset{\overset{\large CH_3}{|}}{N}(CH_2)_qCOOM$$

wherein $R^6$ is $C_{12}$-$C_{18}$ alkyl, M is sodium or potassium and q is 1 or 2.

The examples of the N-acylamino acid and its salt are sodium cocoyl sarcosinate, lauroyl sarcosinate, sodium lauroyl sarcosinate, potassium lauroyl sarcosinate, sodium myristoyl sarcosinate, sodium palmitoyl sarcosinate, oleyl sarcosinate, sodium lauroyl methylalanate and the like. Sodium lauroyl sarcosinate is preferably used.

The polyoxyethylene alkyl ether sulfate used in the present invention is represented by the formula:

$$R^6-O-(CH_2CH_2O)_p-SO_3W$$

wherein $R^6$ is $C_{12}$-$C_{18}$ alkyl, W is sodium, potassium or mono-, di- or tri-ethanolamine and p is an integer of from 6 to 20.

Examples of the polyoxyethylene alkyl ether sulfate are sodium polyoxyethylene (abbreviated to POE hereinafter) (4) lauryl ether sulfate, triethanolamine POE (2) lauryl ether sulfate, triethanolamine POE (3) lauryl ether sulfate, triethanolamine POE (4) lauryl ether sulfate, sodium POE (20) cetyl ether sulfate, triethanolamine POE (20) cetyl ether sulfate and the like. Triethanolamine POE (4) lauryl ether sulfate is preferable.

The fatty acid alkanolamide to be used in the present invention is the one prepared by the reaction of a $C_{12}$-$C_{20}$ fatty acid or its methyl ester with monoethanolamine, diethanolamine or isopropanolamine at the molar ratio of 1:1 or 1:2.
Examples of the fatty acid alkanolamide are monoethanolcocamide, monoethanolstearamide, isopropanollauramide, diethanolcocamide, diethanollauramide, diethanolmyristamide, diethanolstearamide, diethanololeamide or the like in either 1:1 or 1:2 type. As the fatty acid alkanolamide, diethanollauramide (1:1 type) is preferably used.

The water-soluble polyoxyethylene derivative includes polyoxyethylene ether of a $C_{12}$-$C_{18}$ fatty acid ester or an akylphenol with 4 to 20 moles of ethylene oxide, a polyoxyethyleneglycol-type one prepared from a higher fatty acid with 4 to 20 moles of ethylene oxide or a polysorbate-type one of a higher fatty acid sorbitan with 4 to 20 moles of ethylene oxide. They are exemplified as the last one POE (4, 10 or 20) sorbitan monolaurate, POE (6 or 20) sorbitan monooleate, POE (6 or 20) sorbitan monostearate, POE (20) sorbitan trioleate or POE (20) sorbitan tristearate. The prepferaly used water-soluble polyoxyethylene

derivative is POE (20) sorbitan monooleate, POE (6) sorbitan monostearate, POE (20) sorbitan tristearate or the like.

Conditioning agents are a prefarable ingredient and there are exemplified oleiyl alcohol, polyoxyethylene glycol, octyldecanol, isostearic acid, hexyl laurate, isopropyl myristate, myristyl myristate, isopropyl palmitate, butyl stearate, cetyl lactate, myristyl lactate, lanolin and the like.

Representative humectants are propylene glycol, glycerin, polyhydric alcohols and the like.

Represervatives, hair growth stimulants, fragrances, coloring agents, pearl agents and the like are chosen from those conventionally used in cosmetics, especially those allowed to be used by Raw Material Standards for Cosmetics and by Drugs, Cosmetics and Medical Instruments Act. There are exemplified as preservatives, benzoic acid, benzoic acid salts, saicylic acid, salicylic acid salts, parahydroxybenzoic acid esters and the like. They are favorably used in a smaller amount.

A pH adjuster is used to arrange the final pH of the shampoo compositions to about 3 to 8, preferably to weak acidic. Preferable pH adjusters are an inorganic base such as sodium hydroxide or the like and an organic base such as citric acid, triethanolamine, diethanolamine or the like.

The shampoo composition of the present invention can be prepared in the usual manner; for example, a betaine and anionic surfactants are dissolved in a small amount of water while heating and, if desired, a nonionic surfactant with a pearl agent is added thereto

and the mixture is blended. The mixture is, if necessary, further mixed with an emollient agent, a preservative dissolved in a suitable amount of water, a humectant and the like and further blended while heating. Additionally, a hair growth stimulant, fragrances, a coloring agent and others may be added thereto, if desired. The mixture is cooled and its pH is arranged to weak acidic with a pH adjuster. Water is added to the mixture to bring the whole to 100.

The resulting shampoo compositions have low irritation and are applicable to children and adults with sensible skin as well as patients with dermatitis.

The test results of cosmetic performance and comfortability of a shampoo composition of this invention are shown below.

The evaluation parameters are as follows:

(1) Goodness of spreading of the shampoo composition,

(2) Goodness of foaming of the shampoo composition,

(3) Smoothness of the hair while shampooing,

(4) Removability of soil (cleaning effect),

(5) No stiffness of the hair while shampooing,

(6) Ease in rinsing, especially in foaming off,

(7) Ease in rinsing, especially the sliminess caused by residue from the shampoo composition,

(8) Pliability of the hair after shampooing,

(9) No stiffness of the hair after shampooing,

(10) Overall evaluation as to cosmetic performance and confortability, and;

(11) Ease in applying permanent wave.

The evaluation was carried out by the one to one comparison method.

Figure 1 illustrates the results of cosmetic performance and comfortability test with a Shampoo Composition A of this invention (●-●) and commercially available Shampoo Compositions B (○-○) and C (△-△). No difference is found among these three as shown in Figure 1.

The followings are the results of patch test comparing a Shampoo Compositon of this invention (Shampoo A') with commercially available Shampoo D for sensible skin (Shampoo D) and commercially available Shampoo E for tender hair (Shampoo E).

(1) Test Method

To a commercially available Adhesive Plaster For Patch Test® (made by Torii Co.,Ltd, Japan) was applied 0.025 ml each of a 5 % solution of each shampoo. The patches were then placed on the inner side of the upper arm or the forearm (11 female subjects), and removed after 24 hours. Skin irritation was judged by the degree of erythema of the skin after the lapse of 1 hour and 24 hours from removal.

(2) Criteria for Judgement

- – no change
- ㊀ slightly rough (skin squama)
- ± slight erythema
- (±) fairly marked erythema
- \+ marked erythema

(3) Test Results

Table 1

| Time of Judgement | Composition Tested | − | ⊖ | ± | ⊕ | + |
|---|---|---|---|---|---|---|
| after 1 hour | Shampoo A' | 8 | 1 | 2 | | |
| | Sahmpoo D | 5 | 1 | 5 | | |
| | Shampoo E | 5 | | 1 | 3 | 2 |
| after 24 hours | Shampoo A' | 9 | 2 | | | |
| | Shampoo D | 8 | | 3 | | |
| | Shampoo E | 5 | | 3 | 3 | |

As obviously shown in the above test results, the shampoo of the present invention is not inferior in cosmetic performance and comfortability but definitely superior in the patch test to the commercially available shampoos.

The following examples are included merely to aid in the understanding of the invention and variations may be made by one skilled in the art without departing from the spirit and scope of the invention.

Examples

(A) Betaine
Anionic surfactants
deionized water

(B) Nonionic surfactant
Pearl agent

(C) Conditioning agent

Preservative

Humectant

Deionized water

(D) Hair growth stimulant

Fragrance

Coloring agent

pH adjuster

Deionized water

A shampoo composition was prepared with the above components by following the method noted below. The ingredients employed and the amounts are listed in Table 2. The deionized water in (A) and (C) are used in a sufficient amount to dissolve the ingredients listed there.

Preparation method:

Solution (A) prepared at 50 °C is mixed with (B). The resulting mixture is stirred at 50 °C for 10 minutes or at 75 °C if a pearl agent is mixed. (C) is added thereto and stirred and the mixture is maintained at 50 °C (or 75 °C) for 10 minutes and then gradually cooled to 35 °C. (D) is added thereto and the mixture is adjusted to about pH 6.0. Water is added thereto to bring the whole to 100. The composition is gradually cooled to room temperature.

Table 2

| Ingredient | Example No. | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|
| | | Ratio (% by weight) | | | | | | | | | | | | | |
| Betaine | Lauryldimethylammonioacetate | 4.0 | 4.5 | 4.5 | 4.5 | 4.5 | 4.5 | 4.5 | 12.0 | 4.5 | | | | | 10.0 |
| | Cocamidopropyldimethylammonioacetate | | | | | | | | | | 4.6 | 6.0 | | | |
| | Cocamidopropyldimethylammonio-hydroxypropylsulfonate | | | | | | | | | | | | 6.0 | 6.0 | |
| Anionic Surfactant | Triethanolamine Laurylsulfate | 9.0 | 10.4 | 10.4 | 10.4 | 10.4 | 5.5 | 9.0 | 29.0 | 10.4 | 9.0 | 12.6 | 12.6 | 30.0 | 25.0 |
| | Sodium Laurylsulfate | 3.0 | 3.0 | 3.0 | 3.0 | 3.0 | 4.0 | 5.0 | 4.0 | 3.0 | 5.0 | 3.0 | 3.0 | 3.0 | 3.0 |
| | Sodium Lauroylsarcosinate | | | | | | 2.0 | 4.2 | 3.0 | | 3.0 | 2.0 | | 3.0 | 2.0 |
| | Triethanolamine POE (2-4) Lauryl Ether Sulfate | | | | | | | | | 1.8 | | | | | |
| Nonionic Surfactant | Diethaollauramide (1:2 type) | 1.5 | 3.0 | | | | 4.6 | 4.6 | | 4.0 | 4.6 | 6.0 | 6.0 | 12.0 | 6.0 |
| | POE (20) Sorbitan Monooleate | | | 5.0 | | | | | | | | | | | |
| | POE (6) Sorbitan Monostearate | | | | 5.0 | | | | 12.0 | | | | | | 4.0 |
| | POE (20) Sorbitan Tristearate | | | | | 5.0 | | | | | | | | | |
| Emollient Agent | Polyoxyethylene Glycol (300) | 0.25 | | 0.25 | 0.25 | 0.25 | 0.25 | | | 0.25 | | 0.25 | 0.25 | 0.25 | 0.25 |
| | Isostearic Acid | | 0.3 | | | | | 0.3 | 0.3 | | 0.3 | | | | |
| Humectant | Glycerin | 2.0 | | | | | | | | | | | | | |
| Preservative | Methyl Parahydroxybenzoate | 0.02 | The same in the following examples. | | | | | | | | | | | | |
| | Propyl Parahydroxybenzoate | 0.02 | | | | | | | | | | | | | |
| Pearl Agent | POE (1) Monostearate | 1.5 | | | | | | | | | | | | | |
| Coloring Agent | Red No. 106** | * | | | | | | | | | | | | | |
| | Yellow No. ** | * | | | | | | | | | | | | | |
| Fragrance | RG/SHE 961 | * | | | | | | | | | | | | | |
| Hair Growth Stimulant | d-Pantothenol | * | | | | | | | | | | | | | |
| | dl-α-Tocopherol Acetate | * | | | | | | | | | | | | | |
| PH adjustner | Citric Acid | * | | | | | | | | | | | | | |
| Water | Deionized Water | * | | | | | | | | | | | | | |

Notes: * A proper quantity
** Listed in Japanese Standards for Food Additives. Red No. 106 is called Acid Red and Yellow No. 5 corresponds to Yellow No. 6 registered in U.S.A..

Table 2 (Continued)

| Ingredient | Example No. | 15 | 16 | 17 | 18 | 19 | 20 | 21 | 22 | 23 | 24 | 25 | 26 | 27 | 28 |
|---|---|---|---|---|---|---|---|---|---|---|---|---|---|---|---|
| | | Ratio (% by Weight) | | | | | | | | | | | | | |
| Betaine | Lauryldimethylammonioacetate | 10.0 | 6.0 | 8.0 | 10.0 | 6.0 | 6.0 | 6.0 | 7.0 | | | | | 7.0 | 7.0 |
| | Cocamidopropyldimethylammonioacetate | | | | | | | | | 6.9 | 8.4 | | | | |
| | Cocamidopropyldimethylammonio-hydroxypropylsulfonate | | | | | | | | | | | 10.0 | 10.0 | | |
| Anionic Surfactant | Triethanolamine Laurylsulfate | 25.0 | 3.0 | 5.0 | 7.0 | 3.0 | 3.0 | 3.0 | 3.1 | 4.0 | 4.5 | 5.9 | 5.9 | 3.0 | 3.0 |
| | Sodium Laurylsulfate | 3.0 | 3.0 | 3.0 | 3.0 | 3.0 | 3.0 | 3.0 | 3.0 | 3.0 | 3.0 | 3.0 | 3.0 | 2.0 | 2.0 |
| | Sodium Lauroylsarcosinate | 2.0 | | | | | | | 1.0 | | 1.0 | | 1.0 | 2.0 | 2.0 |
| | Triethanolamine POE (4) Lauryl Ether Sulfate | | | | | | | | | | | | | | |
| Nonionic Surfactant | Diethanollauramide | 3.0 | 10.0 | 15.0 | 35.0 | | | | 21.0 | 20.0 | 25.0 | | | 16.0 | 5.0 |
| | POE (20) Sorbitan Monooleate | | | | | 15.0 | | | | | | | | | |
| | POE (6) Sorbitan Monostearate | 7.0 | | | | | 15.0 | | | | | 20.0 | 20.0 | 5.0 | 16.0 |
| | POE (20) Sorbitan Tristearate | | | | | | | 15.0 | | | | | | | |
| Emollient Agent | Polyoxyethylene Glycol (300) | 0.25 | | | | | | | | | | | | | |
| | Isostearic Acid | | | | | | | | | | | | | | |
| Humectant | Glycerin | 2.0 | | | | | | | | | | | | | |
| Preservative | Methyl Parahydroxybenzoate | 0.02 | The same in the following examples. | | | | | | | | | | | | |
| | Propyl Parahydroxybenzoate | 0.02 | | | | | | | | | | | | | |
| Coloring Agent | Red No. 106** | * | | | | | | | | | | | | | |
| | Yellow No. 5** | * | | | | | | | | | | | | | |
| Fragrance | RG/SHE 961 | * | | | | | | | | | | | | | |
| Hair Growth Stimulant | d-Pantothenol | * | | | | | | | | | | | | | |
| | dl-α-Tocopherol Acetate | * | | | | | | | | | | | | | |
| PH adjuster | Citric Acid | * | | | | | | | | | | | | | |
| Water | Deionized Water | * | | | | | | | | | | | | | |

Notes: * A proper quantity
** Listed in Japanese Standards for Food Additives. Red No. 106 is called Acid Red and Yellow No. 5 corresponds to Yellow No. 6 registered in U.S.A..

CLAIMS

1. A low irritating shampoo composition comprising

a. a betaine 1-12 % by weight

b. anionic surfactants consisiting of

(1) triethanolamine laurylsulfate 3-30 % by weight, and

(2) one or more anionic surfactants selected from the group consisting of alkali metal alkylsulfates, N-acylamino acids or its salts and polyoxyethylene alkyl ether sulfonates 1-10 % by weight; and,

c. one or more nonionic surfactants selected form fatty acid alkanolamides and water-soluble polyoxyethylene derivatives 1-36 % by weight

at a weight ratio of about 1:2.5-4:0.6-1 or about 1:1:2.6-3.

2. The composition claimed in claim 1 which comprises about 2-8 % by weight of a betaine, about 5-32 % by weight of two or more anionic surfactants and about 1.2-8 % by weight of one or more nonionic surfactants to the whole amount of the composition.

3. The composition claimed in claim 1 which comprises about 4-10 % by weight of betaine, about 4-10 % by weight of two or more anionic surfactants and about 10.4-30 % by weight of one or more nonionic surfactants to the whole amount of the composition.

4. The composition claimed in claim 1 wherein the betaine is represented by the formula:

$$R^5-\left(\begin{array}{c}\overset{O}{\overset{\|}{-C}}-\overset{R^4}{\overset{|}{N}}-(CH_2)_m-\end{array}\right)_n-\overset{R^2}{\underset{R^3}{\overset{|}{\underset{|}{N^+}}}}-Y-R^1$$

wherein $R^1$ is $COO^-$, $CH_2CH_2SO_3^-$ or $CH(OH)-CH_2SO_3^-$; $R^2$ and $R^3$ are the same or different lower alkyl; $R^4$ is hydrogen or lower alkyl; $R^5$ is higher alkyl; Y is lower alkylene; n is either 0 or 1 and m is an integer of from 2 to 7.

5. The composition claimed in claim 4 wherein the betaine is selected from alkyl betaines, alkylsulfo betaines, alkylamido betaines and alkylamidosulfo betaines.

6. The composition claimed in claim 5 wherein the betaine is lauryldimethylammonioacetate, decyldimethylammonioacatate, cocodimethylammonioacetate, cocodimethylammoniopropylsulfonate, cocamidoethyldimethylammonioacetate, cocamidopropyldimethylammonioacetate, laurylamidopropyldimethylammonioacetate, cocamidopropyldimethylammonioacetate or cocamidopropyldimethylammoniohydroxypropylsulfonate.

7. The composition claimed in claim 6 wherein the betaine is lauryldimethylammonioacetate, cocamidopropyldimethylammonioacetate or cocamidopropyldimethylammoniohydroxypropylsulfonate.

8. The composition claimed in claim 1 wherein the alkylsulfate is represented by the formula:

$$R^6OSO_3M$$

wherein $R^6$ is $C_{12}$-$C_{18}$ alkyl and M is sodium or potassium.

9. The composition claimed in claim 8 wherein the alkali metal alkylsulfate is sodium laurylsulfate, potassium laurylsulfate, sodium myristylsulfate, sodium cetylsulfate or sodium stearylsulfate.

10. The composition claimed in claim 9 wherein the alkali metal alkylsulfate is sodium laurylsulfate.

11. The composition claimed in claim 1 wherein the N-acylamino acid and its salt are represented by the formula.

$$R^6\text{-}\overset{\overset{O}{\|}}{C}\text{-}\overset{\overset{CH_3}{|}}{N}(CH_2)_qCOOM$$

wherein $R^6$ is $C_{12}$-$C_{18}$ alkyl, M is sodium or potassium and q is 1 or 2.

12. The composition claimed in claim 11 wherein the N-acylamino acid is sodium cocoyl sarcosinate, lauroyl sarcosinate, sodium lauroyl sarcosinate, potassium lauroyl sarcosinate, sodium myristoyl sarcosinate, sodium palmitoyl sarcosinate, oleyl sarcosinate or sodium lauroyl methylalanate.

13. The composition claimed in claim 12 wherein the N-acylamino acid is sodium lauroyl sarcosinate.

14. The composition claimed in claim 1 wherein the

polyoxyethylene alkyl ether sulfate is represented by the formula:

$$R^6\text{-O-}(CH_2CH_2O)_p\text{-}SO_3W$$

wherein $R^6$ is $C_{12}$-$C_{18}$ alkyl, W is sodium, potassium or mono-, di-or triethanolamine and p is an integer of from 6 to 20.

15. The composition claimed in claim 14 wherein the polyoxyethylene alkyl ether sulfate is sodium POE (4) lauryl ether sulfate, triethanolamine POE (2) lauryl ether sulfate, triethanolamine POE (3) lauryl ether sulfate, triethanolamine POE (4) lauryl ether sulfate, sodium POE (20) cetyl ether sulfate or triethanolamine POE (20) cetyl ether sulfate.

16. The composition claimed in claim 15 wherein the polyoxyethylene alkyl ether sulfate is triethanolamine POE (4) lauryl ether sulfate.

17. The composition claimed in claim 1 wherein the fatty acid alkanolamide is the one prepared by the reaction of a $C_{12}$-$C_{20}$ fatty acid or its methyl ester with monoethanolamine, diethanolamine or isopropanolamine at the molar ratio of either 1:1 or 1:2.

18. The composition claimed in claim 17 wherein the alkanolamide is monoethanolcocamide, monoethanolstearamide, isopropanollauramide, diethanolcocamide, diethanollauramide, diethanolmyristamide, diethanolstearamide or diethanololeamide in either 1:1 or 1:2 type.

19. The composition claimed in claim 18 wherein the alkanolamide is diethanollauramide (1:1 type).

20. The composition claimed in claim 1 wherein the water-soluble polyoxyethylene derivative is a polyoxyethylene ether of a $C_{12}$-$C_{18}$ fatty acid ester or an akylphenol with 4 to 20 moles of ethylene oxide, a polyoxyethyleneglycol-type one of a higher fatty acid with 4 to 20 moles of ethylene oxide or a polysorbate-type one of a higher fatty acid sorbitan with 4 to 20 moles of ethylene oxide.

21. The composition claimed in claim 20 wherein the water-soluble polyoxyethylene derivative is POE (4, 10 or 20) sorbitan monolaurate, POE (6 or 20) sorbitan monooleate, POE (6 or 20) sorbitan monostearate, POE (20) sorbitan trioleate or POE (20) sorbitan tristearate.

22. The composition claimed in claim 21 wherein the water-soluble polyoxyethylene derivative is POE (20) sorbitan monooleate, POE (6) sorbitan monostearate or POE (20) sorbitan tristearate.

1/1

Figure 1

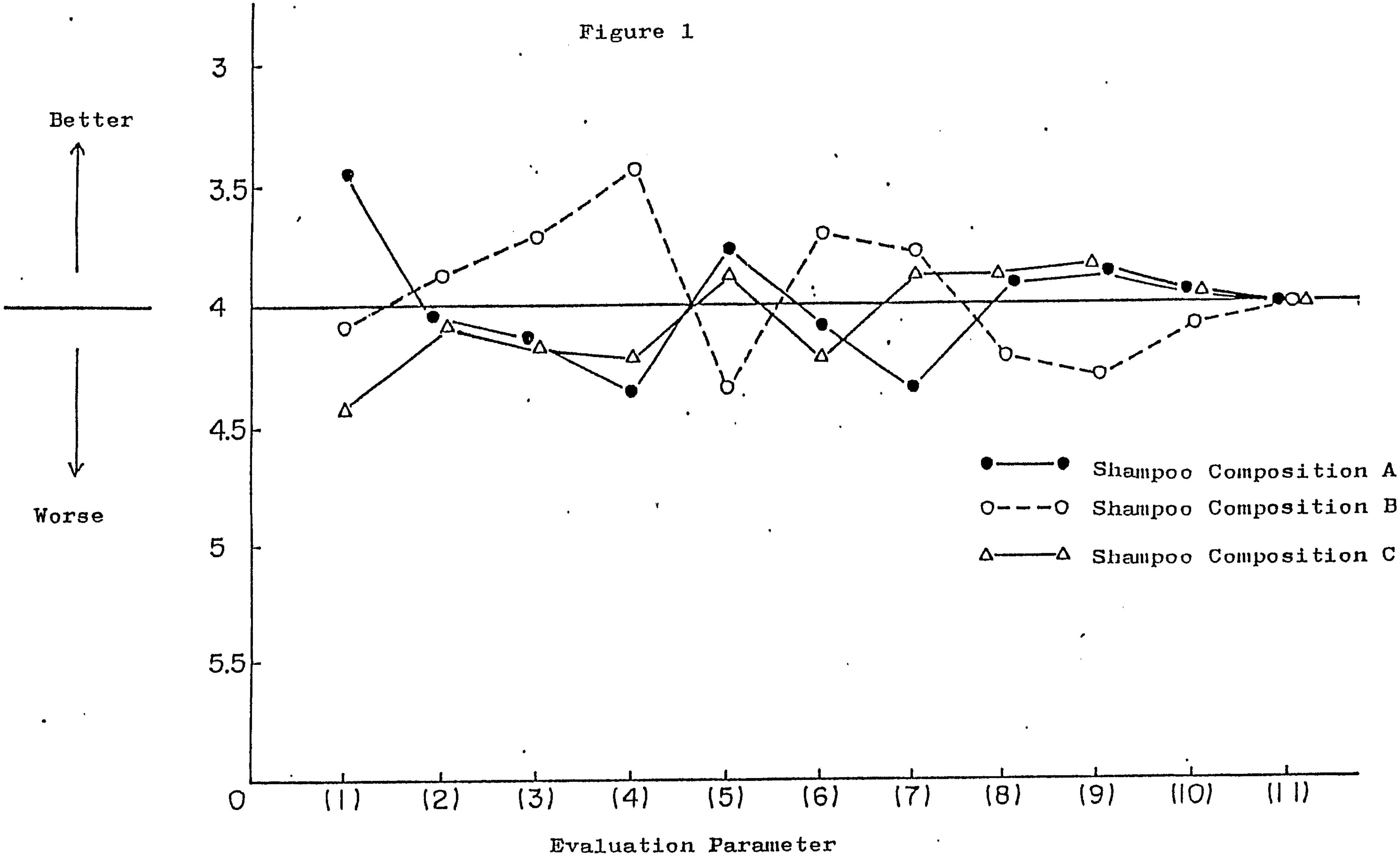
Better
Worse
3
3.5
4
4.5
5
5.5
0
(1)
(2)
(3)
(4)
(5)
(6)
(7)
(8)
(9)
(10)
(11)
Evaluation Parameter
Shampoo Composition A
Shampoo Composition B
Shampoo Composition C